# EUROPEAN PATENT APPLICATION

(11) **EP 4 725 417 A1**
(43) Date of publication of application: **15.04.2026**
(21) Application number: 24383098.1
(22) Date of filing: 09.10.2024
(51) Int. Cl.: A61B 5/263, A61B 5/293, A61B 5/00

(54) **NEURAL PROBE AND MANUFACTURING METHOD THEREOF**

(71) Applicant: Fundació Institut Català de Nanociència i Nanotecnologia (ICN2), 08193 Bellaterra (ES); Fundació Institució Catalana de Recerca I Estudis Avançats (ICREA), 08010 Barcelona (ES); Consejo Superior De Investigaciones Científicas - CSIC, 28006 Madrid (ES); Consorcio Centro de Investigación Biomédica en Red (CIBER), 28029 Madrid (ES); UCL Business Ltd, London Greater London WC1E 6BT (GB)
(72) Inventor: Garrido Ariza, José Antonio, 08960 Sant Just Desvern, Barcelona (ES); Masvidal Codina, Eduard, 08193 Cerdanyola del Vallès (Barcelona) (ES); Guimerà Brunet, Antón, 08193 Cerdanyola del Vallès, Barcelona (ES); Prokop, Michal, 08193 Barcelona (ES); Garcia Cortadella, Ramon, Creixell (Tarragona) (ES); Illa Vila, Xavier, 28029 Madrid (ES); Esparza Iaizzo, Martin, London (GB); Wykes, Rob, London (GB); Codadu, Neela, London (GB)
(74) Representative: DTS Patent- und Rechtsanwälte PartmbB

(57) **Abstract**

The present invention provides a stimulation and recording neural probe (100) at least comprising: a substrate (10); a stimulation and recording array (12), said stimulation and recording array (12) including at least one single-layer or double-layer solution-gated graphene field effect transistor (SG-GFET) (14) for recording brain signals of a patient, and at least one electrode (16) for applying electrical stimulation to a brain tissue and/or recording brain signals of the patient; and metal tracks (18, 20) for the at least one electrode (16) and the at least one single-layer or double layer SG-GFET (14), encapsulated in respective first and second encapsulation layers (22, 24). The invention further provides a method of manufacturing a stimulation and recording neural probe, such as the neural probe described above. Still further, the invention provides closed-loop system (200) for brain stimulation and recording comprising a control unit (202) and a stimulation unit (204), the stimulation unit (204) comprising at least one neural probe as described above.

## Description

The present invention belongs to the technical field of devices, systems, and techniques for delivery of therapeutic electrical stimulation to a tissue, especially brain tissue, of a patient in need thereof.

In particular, the present invention provides a neural probe, e.g., a flexible neural probe, for recording brain signals and delivery of electrical stimulation to a brain tissue of a patient in need thereof.

The present invention further provides a method of manufacturing a stimulation and recording neural probe, such as the neural probe defined above.

Still further, the invention provides a stimulation system, inter alia including at least one neural probe as defined above.

The system is adapted for delivery of closed-loop electrical stimulation to a target tissue within the brain of a patient.

Neural probes for brain stimulation and recording are known in the art.

In general, neural probes of this kind are adapted for brain implantation, and include one or more electrodes for applying electrical stimulation to a target tissue.

Also, neural probes may be configured to record brain signals (e.g., through one or more recording electrodes).

Accordingly, electrical stimulation can be inter alia delivered in a closed-loop mode.

That is, electrical stimulation can be triggered and/or controlled and/or adjusted based on one or more detected signals, e.g. brain signals, from a patient.

In general, one or more electrodes can be adapted to both record brain signals and apply electrical stimulation.

Also, the use of transistors allows for recording brain signals with improved reliability.

Nevertheless, said known neural probes are still sub-optimal.

In particular, currently utilized neural probes have proven unable to detect infraslow (<0.1 Hz) brain activity, thus ignoring this important part of the spectrum.

This means that certain neurological disorders, which biomarkers have proven to demonstrate themselves in infraslow frequencies, cannot be successfully treated.

Neurological disorders of this kind inter alia include epilepsy or stroke.

At present, such neurological disorders affect millions of people worldwide.

Therefore, there is a strongly-felt need for an improved solution allowing successfully treating a wider range of neurological disorders, including those characterized by biomarkers demonstrating themselves in infraslow frequencies, e.g. epilepsy or stroke.

Additionally, currently-utilized neural probes still do not allow obtaining reliable recording of brain signals during stimulation, because of the persisting stimulation artifact obscuring underlying bio signal.

In view of the above, it is an object of the present invention to provide a stimulation and recording flexible neural probe capable of recording full frequency spectrum of brain signals, and use this information to trigger and/or control and/or adjust applied brain stimulation, e.g. in a closed-loop fashion.

The above-mentioned object is obtained by the provision of a stimulation and recording flexible neural probe as defined in claim 1.

According to the invention, a stimulation and recording probe at least comprises:
a substrate;
a stimulation and recording array comprising:
   at least one single-layer or double layer solution-gated graphene field effect transistor (SG-GFET) for recording brain signals of a patient, and
   at least one electrode for applying electrical stimulation to a brain tissue and/or recording brain signals of the patient, and
metal tracks for the at least one electrode and the at least one single-layer or double layer SG-GFET, encapsulated in respective first and second encapsulation layers.

The present invention provides a stimulation and recording neural probe.

The neural probe comprises a substrate.

The neural probe further comprises a stimulation and recording array.

The stimulation and recording array comprises at least one solution-gated graphene field effect transistor (SG-GFET).

In particular, the stimulation and recording array may comprise at least one single-layer SG-GFET.

Alternatively, the stimulation and recording array may comprise at least one double-layer SG-GFET.

For instance, the array may include a plurality of single-layer or double layer SG-GFETs.

The at least one single-layer or double layer SG-GFET is configured for recording brain signals of a patient.

The stimulation and recording array further comprises at least one electrode.

For instance, the array may include a plurality of electrodes.

The at least one electrode is configured for applying electrical stimulation to a target tissue within the brain of the patient.

Additionally, the at least one rGO electrode may be configured for recording brain signals of the patient.

In general, the same electrode may be adapted to both apply electrical stimulation and record brain signals.

The neural probe further comprises metal tracks for the at least one single-layer or double layer SG-GFET and the at least one electrode.

SG-GFETs allow avoiding drift and signal attenuation, thereby overcoming the drawbacks of known metal-based recording electrodes. Additionally, due to intrinsic signal preamplification and voltage to current conversion, SG-GFETs allow for reliable signal recording even during electrical stimulation.

Hence, recording of brain activity is enabled in the full frequency spectrum (i.e. including the infraslow frequency range), and at all times (including during or right after stimulation).

That is, the neural probe of the invention addresses the need of recording full frequency spectrum of brain signals and overcomes the challenge of signal recording during or right after stimulation. These features are crucial for the new generation of closed-loop systems for neurological disorders treatment. The invention is based on the basic idea that, by the provision of a stimulation and recording array combining SG-GFETs and stimulation and recording electrodes, bidirectional neural interfacing and stimulation control (e.g. in closed-loop fashion) based on recording of the whole frequency range of brain signals can be enabled, at the same time preventing stimulation artifact.

Thanks to the ability of SG-GFETs to detect infraslow (<0.1Hz) brain activity, the neural probe of the invention can be used for treatment of a wide range of pathologies, including those neurological disorders the biomarkers of which demonstrate themselves in infraslow frequencies.

The neural probe of the invention combines brain stimulation and recording capabilities, and is thus adapted for use in stimulation systems (inter alia) operating in a closed-loop mode.

Here, a predefined stimulation protocol can be triggered and/or controlled and/or adjusted based on a biomarker (even when hidden in the infraslow activity range) detected and recorded by the one or more SG-GFETs.

Additionally, inherent multiplexing properties of the SG-GFETs have a potential for high-density recordings, unobtainable using unimodal electrode-based configurations.

Advantageously, the at least one electrode can be a reduced-graphene oxide (rGO) electrode.

rGO electrodes are characterized by a porous and flexible design with high-charge injection capacity.

Accordingly, safe and efficient brain tissue stimulation can be provided.

An amorphous rGO film suitable for use in the neural probe of the invention is described in EP3702327A1, filed in the name of the same applicant. This known rGO film has a total thickness from 20 nm to 5 micrometers, and comprises pores characterized by a diameter equal to or less than 10 nm.

Electrochemical inertness, biocompatibility, and excellent mechanical properties render graphene-based solutions particularly convenient, especially in medical applications.

Alternatively, other kinds of electrodes, such as metal, e.g., platinum, electrodes, metal oxides, e.g. iridium oxide, electrodes, or PEDOT:PSS electrodes can also be used according to the invention.

Advantageously, the substrate, the first encapsulation layer, and the encapsulation second layer can be made of a flexible material.

Accordingly, a flexible neural probe can be provided, which better adapts to the anatomy of the patient.

For example, the first and second encapsulation layers can be made of a polymeric material.

Alternatively, the first and second encapsulation layers can be made of a non-polymeric material.

As a further alternative, the first and second encapsulation layers can be made of a combination of a polymeric and non-polymeric material.

As a non-limiting example, the substrate as well as the first and second encapsulation layers can be made of Polyimide.

As a further example, the substrate and the first and second encapsulation layers can be made of Parylene-C.

However, the use of different materials is also possible, provided that they are suitable for the purpose.

Preferably, the stimulation and recording array comprises a plurality of single-layer or double layer SG-GFETs and electrodes, e.g. rGO electrodes.

Preferably, the stimulation and recording array comprises an arrangement including rows of single-layer or double layer SG-GFETs alternated with rows of electrodes.

Accordingly, stimulation and recording can be implemented with enhanced accuracy.

For instance, the stimulation and recording array may comprise 16 single-layer or double layer SG-GFETs and 14 electrodes.

In this configuration, the stimulation and recording array may comprise an arrangement including four rows of single-layer or double layer SG-GFETs alternated with four rows of electrodes.

In this configuration, the bottom row of electrodes may include a reduced number of electrodes.

For instance, the bottom row of electrodes may include two electrodes.

Otherwise stated, in this configuration, each row of single-layer or double layer SG-GFETs may include 4 single-layer or double layer SG-GFETs, and each row or electrodes may include 4 electrodes, with the exception of the row formed at the bottom of the array, where two electrodes are removed.

However, in principle, the choice regarding the number of single-layer or double layer SG-GFETs and electrodes, as well as their arrangement, shall be carried out on a case-by-case basis, based on the current therapeutic needs.

Advantageously, the at least one single-layer or double layer SG-GFET may have a channel length between 1 × 1 µm to 1 × 1 mm.

Preferably, the at least one single-layer or double layer SG-GFET has a channel length of 50 x 50 µm.

Advantageously, the at least one electrode may have a diameter between 10 µm to 5 mm.

Preferably, the at least one electrode has a diameter of 100 µm.

Advantageously, the stimulation and recording array can be provided at a tip portion of the neural probe.

The neural probe may further comprise an additional top layer.

This additional layer allows providing further protection for the stacked components of the neural probe.

Advantageously, said additional top layer can be made of SU-8.

SU-8 is a commonly used epoxy-based negative photoresist.

The invention further provides a method of manufacturing a stimulation and recording neural probe.

Conveniently, the method can be implemented for manufacturing the neural probe described in the foregoing.

The method at least comprises the following steps of:
providing a substrate
forming a first encapsulation layer on said substrate;
forming a first metal layer for electrode tracks on the first encapsulation layer;
defining and patterning at least one and the electrode metal tracks;
forming a second flexible encapsulation layer;
defining and patterning VIAs;
defining and patterning the at least one single-layer or double layer SG-GFET and SG-GFET metal tracks;
defining and patterning openings for the at least one electrode, and
defining and patterning an outline for the neural probe.

A sacrificial substrate, e.g., a Si/SiO2 wafer, may be used during manufacturing of the probe.

This provides support to the flexible substrate during manufacturing.

The use of a rigid sacrificial substrate, e.g., a Si/SiO2 wafer is advantageous when manufacturing a flexible neural probe to provide sufficient support.

Upon completion of the manufacturing process, the obtained probe is detached from the sacrificial substrate.

For instance, a photolithography process can be implemented to define the shape of the SG-GFET metal tracks.

Then, metal is evaporated on the defined tracks.

Eventually, a lift-off process is implemented to remove excess of metal.

Advantageously, the method may further comprise at least one passivation step.

Said passivation step may include interlayer passivation.

Additionally or alternatively, said passivation step may include top passivation.

Additionally or alternatively, said passivation step may include bottom passivation.

The method may further comprise a step of forming a second metal layer for at least one single-layer or double layer solution-gated graphene field effect transistor (SG-GFET) over the second flexible encapsulation layer.

Then, a graphene preparation is prepared and transferred on the second metal layer.

As a non-limiting example, these additional steps can be implemented after patterning of the VIAs.

Advantageously, the at least one electrode can be a reduced-graphene oxide (rGO) electrode.

In such a case, before the step of defining and patterning at least one electrode and the electrode metal tracks, the method further comprises the steps of:
filtrating a graphene oxide dispersion;
forming and transferring a graphene oxide membrane over the first metal layer.

Alternatively, other kinds of electrodes, such as metal, e.g., platinum, electrodes, metal oxide, e.g. iridium oxide, electrodes, or PEDOT:PSS electrodes can also be used according to the invention. Advantageously, the substrate, the first encapsulation layer, and the second encapsulation layer can be made of a flexible material.

For example, the first and second encapsulation layers can be made of a polymeric material.

Alternatively, the first and second encapsulation layers can be made of a non-polymeric material.

As a further alternative, the first and second encapsulation layers can be made of a combination of a polymeric and non-polymeric material.

As a non-limiting example, the substrate as well as the first and second encapsulation layers can be made of Polyimide.

As a further example, the substrate and the first and second encapsulation layers can be made of Parylene-C.

However, the use of different materials is also possible, provided that they are suitable for the purpose. The method may further comprise providing an additional top layer.

Preferably, said additional top layer is made of SU-8.

Still further, the present invention provides a system for brain stimulation and recording.

In particular, the system is adapted for delivery of closed-loop stimulation.

The system comprises a control unit.

Also, the system comprises a stimulation unit.

The stimulation unit is operatively connected to the control unit.

That is, operation of the stimulation unit can be controlled by the control unit.

The stimulation unit comprises at least one stimulation and recording neural probe as described in the foregoing.

Accordingly, electrical stimulation can be triggered and/or controlled and/or adjusted based on one or more brain signals of the patient, detected and recorded through the at least one single-layer or double layer SG-GFET.

Further details and advantages of the invention shall now be disclosed in connection with the drawings, where:

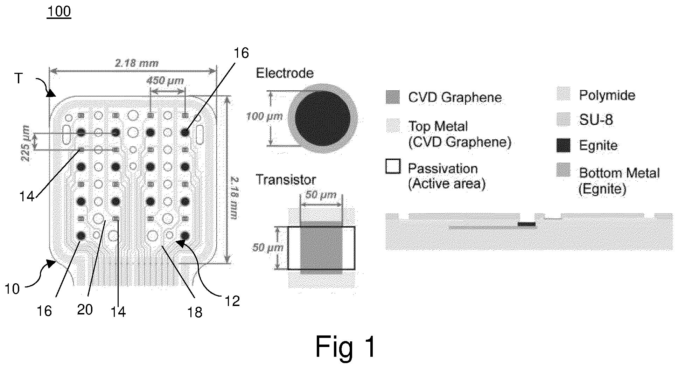

- **Fig. 1**: shows a stimulation and recording neural probe, here a flexible neural probe, according to an embodiment of the invention. Here, the probe comprises a stimulation and recording array including 16 single-layer SG-GFETs and 14 electrodes, here rGO electrodes, arranged in alternating rows;

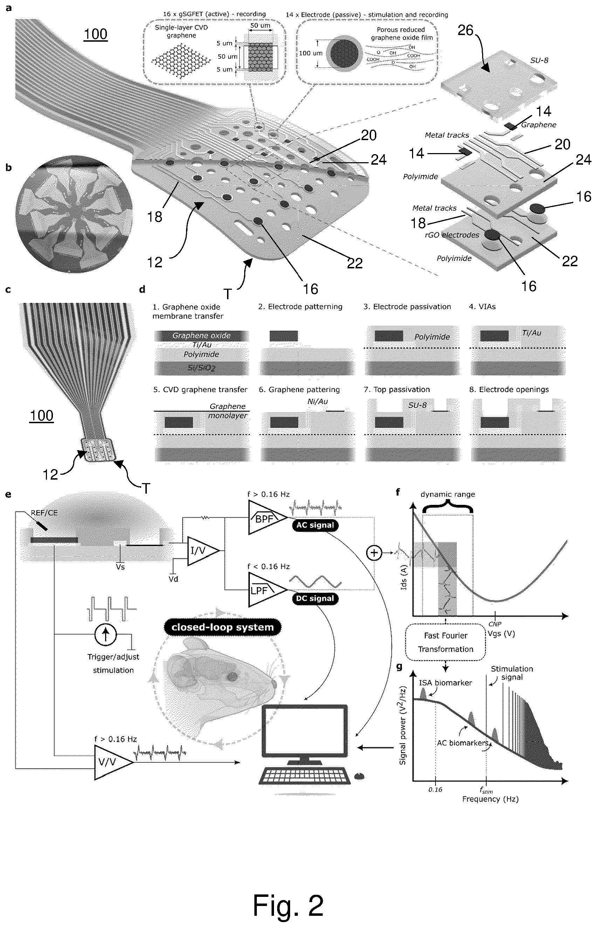

- **Figs. 2a-g**: show details and functionalities of the neural probe of **Fig. 1****.** In detail: **a.** perspective and exploded views of the neural probe of **Fig**. **1****.; b.** 4" wafer for use as sacrificial substrate for the fabrication of the probe 100; **c.** different view of the stimulation and recording flexible neural probe of **Fig. 1****; d.** schematic and simplified overview of a method of manufacturing the neural probe of **Fig. 1****; e.** schematic overview of the functioning of a closed-loop stimulation and recording system, inter alia including at least one neural probe as shown in **Fig. 1****.** In particular, the diagram shows an experimental implementation in a rodent (mice) model; f. schematic representation of current to voltage conversion in a single-layer SG-GFET; **g.** full-bandwidth acquisition capabilities in a single-layer SG-GFET;

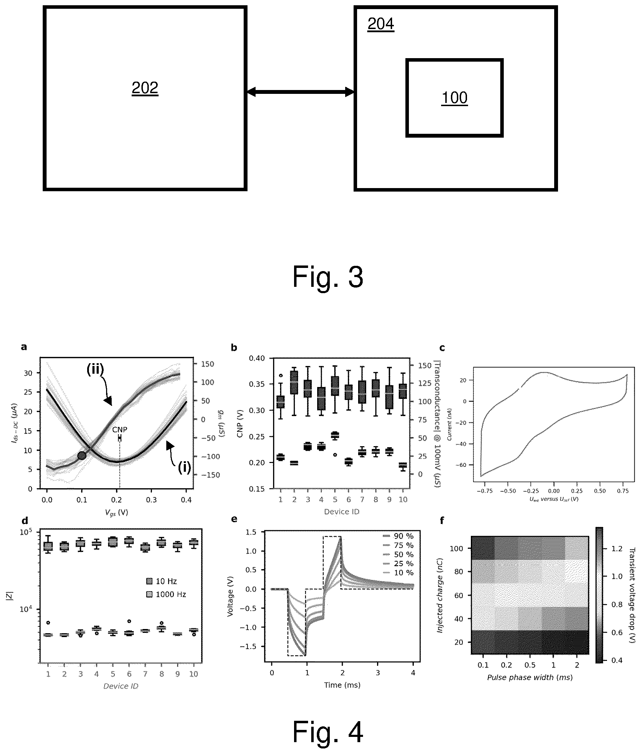

- **Fig. 3**: is a block diagram showing a system, in particular a closed-loop system, for brain stimulation and recording, inter alia including a stimulation and recording neural probe, here a flexible neural probe, according to the invention, e.g. as shown in **Fig. 1**.
- **Figs. 4a-f**: show characterization of a neural probe according to the invention, e.g., as shown in **Fig**. **1**. In detail: **a.** individual transfer curves (curves (i)) and transconductance (curves **(ii))** of 16 single-layer SG-GFETs in one device. Here, thicker lines are averages. **b.** CNP and transconductance values (for Vgs = CNP - 0.1V) dispersion statistics for 10 devices (16 transistors each). **c.** cyclic voltammetry measurement in [-0.8, 0.8 V] range, 50mV/s rate. **d.** impedance values at 10 and 1000 Hz for 10 devices (14 rGO electrodes each). **e.** voltage response to current-controlled biphasic pulses of 500 us per phase (dashed lined) applied through rGO electrodes, 5 pulse amplitudes defined as a percentage of Amax = 210 µA. **f.** map of the cathodic capacitive voltage excursion occurring at the interface between rGO electrodes and the electrolyte during the injection of current pulses at different levels of injected charge and pulse widths.

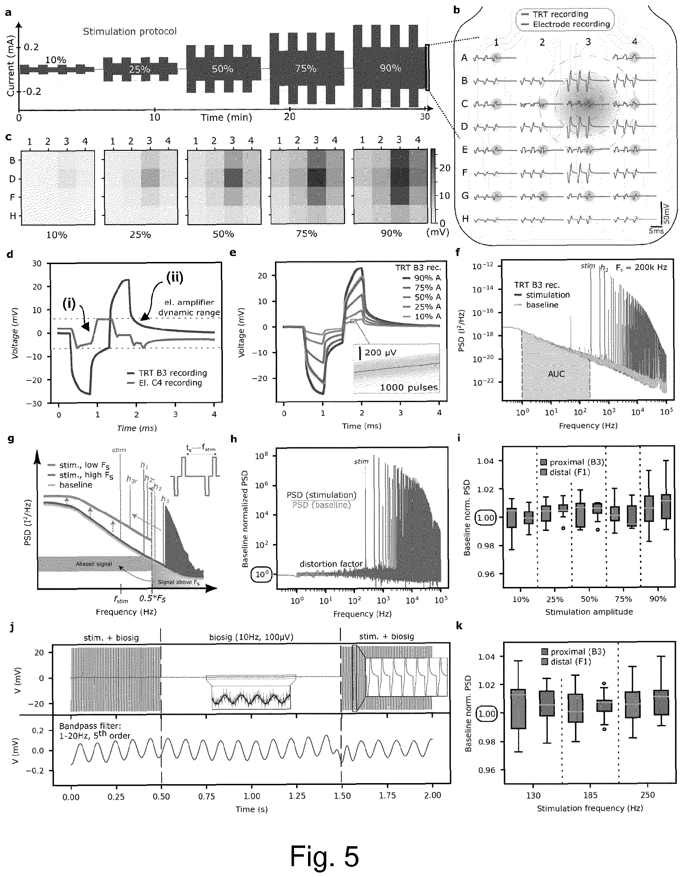

- **Figs. 5a-k**: show signal recording performance for the probe during simultaneous stimulation with rGO electrodes. In detail: **a.** stimulation protocol used in the experiments with combinations of various stimulation parameters: pulse width (100 or 500 µs per phase), frequency (25, 130, 185 or 250 Hz), amplitude (10, 25, 50, 75 or 90 % Amax). Each combination lasts -40s. **b.** electrode ( line (i)) and transistor (line **(ii))** recordings. Mapping of the last 3 pulses (250 Hz, 500 µs/phase, 90% A) in the stimulation protocol. Stimulation through electrode C3. **c.** voltage mapping by transistors. **d.** comparison of 500 µs pulse recorded by a transistor and electrode showing electrode amplifier saturation **e.** transistor recording, 1000 stimulation pulses (F=250 Hz, t=500 µs, A=90%) overlapped. **f.** PSD plot of recording of a baseline and a stimulation period (F=250 Hz, t=500 µs, A=90%). **g.** schematic of noise aliasing and signal quality dependence on sampling frequency. **h.** ratio of PSD plots from **f.. i.** statistics of baseline normalized PSD value dependency on stimulation amplitude. 9 full stimulation protocols from **a.** averaged. **j.** raw recording and recovery of 10Hz biosignal during stimulation. **k.** same as in **i.,** but as a function of stimulation frequency.

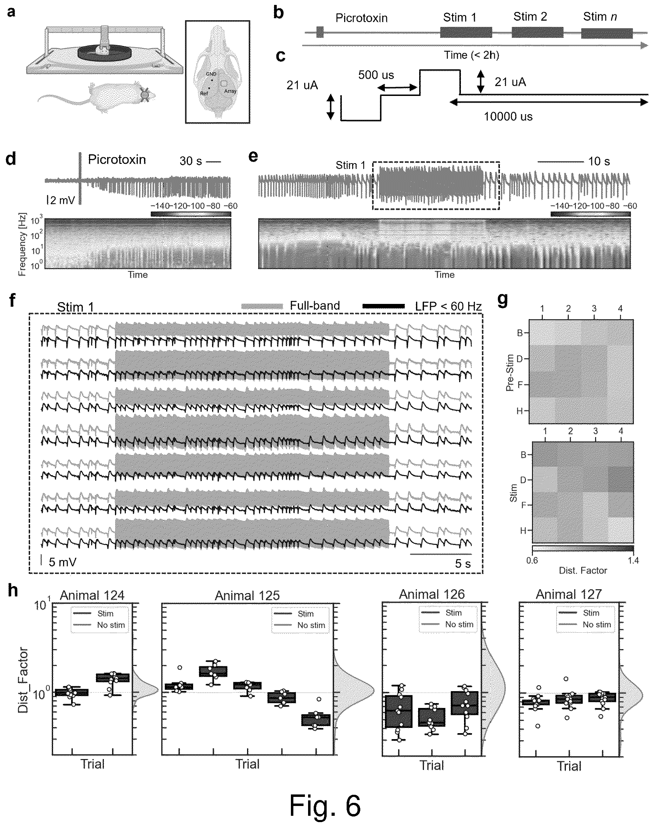

- **Figs. 6a-h**: *In vivo* stimulation artifact characterization; In detail: **a.** schematic drawing of mouse on Neurotar head-fixation system with a blow up of the top cranial view showing the hybrid array position relative to ground and reference wires. **b.** experimental timeline with indication of the picrotoxin chemo convulsant injection and subsequent stimulations. **c.** detail of delayed biphasic stimulation protocol. **d-e**. voltage recording and time-frequency analysis of an example transistor channel before and after picrotoxin injection (d) and before after electrical stimulation. Note the limited spread of stimulation bands leaving biological activity of lower frequency unaffected. **f.** blow-up of the first stimulation period across multiple channels with full-band (gray) and low-pass filtered at 60Hz activity (black). **g.** spatial colormap of the distortion factor before and during stimulation for all transistor channels. **h.** distortion factor across consecutive trials of 4 animals with a kernel density estimate of the surrogate values in the absence of stimulation.

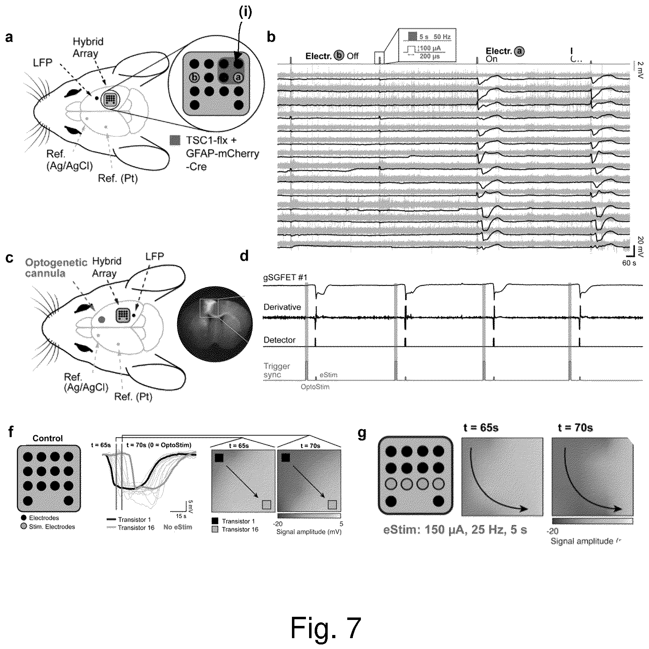

- **Figs. 7a-g**: Proof of concept of *in vivo* applications. **a.** detail of focal epileptic mouse model with injection site of TSC1 knock out agent. Here, the mouse was implanted with a neural probe according to the invention, e.g., as illustrated in **Fig. 1****.** Emphasized area denoted with **(i)** is putatively hyperexcitable. **b.** demonstration of the hybrid array to map epileptiform tissue with stimulation off the hyperexcitable site not eliciting responses while stimulation over the excitable site does. **c.** detail of wild-type mice with channelrhodopsin injection to make the mouse reactive to optic stimulation. **d.** demonstration of reliable induction of infraslow activity with optogenetics, detection with the single-layer SG-GFET, and stimulation with rGO electrodes, **f.** proof of propagation mapping of infraslow activity across the device, and **g.** the resulting change in direction due to stimulation in a row of electrodes

**Fig.** 1 schematically shows a stimulation and recording neural probe 100 according to an embodiment of the invention.

Further details of the neural probe 100 are shown in **Figs. 2a** and **2c****.**

In the shown embodiment, the neural probe 100 is flexible.

Not shown is that the neural probe 100 can also be rigid.

The neural probe 100 comprises a substrate 10 (not shown in **Fig. 2a****).**

In the present embodiment, the substrate 10 is flexible.

As a non-limiting example, the flexible substrate 10 can be made of Polyimide.

As a further example, the flexible substrate 10 can be made of Parylene-C.

However, the use of different flexible materials is also possible, provided that they are suitable for the purpose.

The neural probe 100 further comprises a stimulation and recording array 12.

In the present embodiment, the stimulation and recording array 12 comprises at least one single-layer solution-gated graphene field effect transistor (SG-GFET) 14 for recording brain signals of a patient.

Not shown is that, alternatively, the stimulation and recording array 12 may comprise at least one double-layer solution-gated graphene field effect transistor (SG-GFET)14.

In the present embodiment, the stimulation and recording array 12 comprises a plurality of single-layer SG-GFETs 14 **(****Figs. 1** and **2a****).**

As mentioned, single-layer SG-GFETs allow for recording brain activity in the full frequency spectrum, including the infraslow (<0.1Hz) frequency range.

The stimulation and recording array 12 further comprises at least one electrode 16.

In the present embodiment, the at least one electrode is reduced-graphene oxide (rGO).

Not shown is that other kinds of electrodes, such as metal, e.g., platinum, electrodes, or PDOT:PSS electrodes can also be used according to the invention.

In particular, int the present embodiment, the stimulation and recording array 12 comprises a plurality of rGO electrodes 16 **(****Figs. 1** and **2a****).**

The rGO electrodes 16 are configured for applying electrical stimulation to a target tissue within the brain of a patient.

The rGO electrodes may be also configured for recording brain signals of the patient.

In particular, the same rGO electrode 16 can be used for both stimulation and recording.

rGO electrodes are characterized by a porous and flexible design and high-charge injection capacity.

Accordingly, safe and efficient brain tissue stimulation can be provided.

The neural probe 100 further comprises metal tracks 18, 20 for the rGO electrodes 16 and the single-layer SG-GFETs 14 **(****Fig. 2a****).**

As shown in detail in the exploded view of **Fig. 2a****,** said metal tracks 18, 20 are respectively encapsulated in respective first and second encapsulation layers 22, 24.

In the present embodiment, the first and second encapsulation layers 22, 24 are flexible.

In the present embodiment, the first and second encapsulation layers 22, 24 are made of a polymeric material.

As a non-limiting example, the first and second encapsulation layers 22, 24 can be made of Polyimide.

As a further example, the first and second encapsulation layers 22, 24 can be made of Parylene-C.

However, different polymer materials can also be used according to the invention, provided that they are suitable for the purpose.

Alternatively, the first and second encapsulation layers 22, 24 can be made of a non-polymeric material.

As a further alternative, the first and second encapsulation layers 22, 24 can be made of a combination of a polymeric and non-polymeric material.

In the present embodiment, the neural probe further comprises an additional top layer 26.

In particular, in the present embodiment, said additional top layer 26 is made of SU-8.

As mentioned, SU-8 is a commonly used epoxy-based negative photoresist.

In the present embodiment, the stimulation and recording array 12 comprises an arrangement including rows of single-layer SG-GFETs 14 alternated with rows of rGO electrodes 16 **(****Figs. 1** and **2a****).**

In the present embodiment, as shown in **Fig. 1**, the stimulation and recording array 12 comprises 16 single-layer SG-GFETs 14 and 14 rGO electrodes 16.

In particular, in the present embodiment, the stimulation and recording array 12 comprises an arrangement including four rows of single-layer SG-GFETs 14 alternated with four rows of rGO electrodes 16 **(****Figs. 1** and **2a****).**

The last row of rGO electrodes 16, formed at the bottom of the array 12, includes a reduced number of rGO electrodes 16.

Here, in particular, said bottom row of rGO electrodes 16 includes two electrodes16 (**Fig. 1**).

Otherwise stated, each row of single-layer SG-GFETs 14 comprises 4 single-layer SG-GFETs 14, and each row of rGO electrodes 16 includes 4 rGO electrodes 16, with the exception of the bottom row of rGO electrodes 16 that only includes two rGO electrodes 16 **(****Figs. 1** and **2a****).**

Not shown is that other configurations for the stimulation and recording array 12 are also possible according to the invention.

In particular, the choice regarding the number of single-layer SG-GFETs 14 and rGO electrodes 16, as well as their arrangement, shall be carried out on a case-by-case basis, based on the current therapeutic needs.

In general, the at least one single-layer SG-GFET 14 may have a channel length between 1 x 1 µm to 1 x 1 mm.

In the present embodiment, the at least one single-layer SG-GFET 14 has a channel length of 50 x 50 µm.

In general, the at least one rGO electrode 16 may have a diameter between 10 µm to 5 mm.

In the present embodiment, the at least one rGO electrode 16 has diameter of 100 µm.

Advantageously, the stimulation and recording array 12 is provided at a tip portion T of the neural probe 10.

The present invention further provides a method of manufacturing a stimulation and recording neural probe.

Conveniently, the method can be implemented for manufacturing the neural probe 100 described in the foregoing.

A simplified overview of the method is schematically provided in **Fig. 2d****.**

In particular, the method at least comprises:
providing a substrate 10;
forming a first encapsulation layer 22 on said substrate 10;
forming a first metal layer for electrode tracks 18 on the first encapsulation layer 22;
defining and patterning at least one electrode 16 and the electrode metal tracks 18;
forming a second encapsulation layer 24;
defining and patterning VIAs;
defining and patterning the at least one single-layer or double-layer SG-GFET 14 and SG-GFET metal tracks 20;
defining and patterning openings for the at least one electrode 16, and
defining and patterning an outline for the neural probe 100.

In the present embodiment, the at least one electrode 16 is a rGO electrode 16.

Accordingly, before the step of defining and patterning at least one electrode 16 and the electrode metal tracks 18, the method further comprises the steps of:
filtrating a graphene oxide dispersion;
forming and transferring a graphene oxide membrane over the first metal layer.

Not shown is that other kinds of electrodes, such as metal, e.g., platinum, electrodes, metal oxide, e.g. iridium oxide, electrodes, or PEDOT:PSS electrodes can also be used according to the invention.

In the present embodiment, the neural probe 100 is flexible.

Accordingly, the substrate 10 as well as the first and second encapsulation layers 22, 23 are made of a flexible material.

In the present embodiment, the first and second encapsulation layers 22, 24 are made of a polymeric material.

As a non-limiting example, Polyimide and Parylene C can be used as suitable materials for the substrate 10 and the first and encapsulation polymer layers 22, 23.

However, the use of different polymeric materials is also possible, provided that they are suitable for the purpose.

Alternatively, the first and second encapsulation layers 22, 24 can be made of a non-polymeric material.

As a further alternative, the first and second encapsulation layers 22, 24 can be made of a combination of a polymeric and a non-polymeric material.

A sacrificial substrate, e.g., a Si/SiO2 wafer, may be used during manufacturing of the probe 100.

The use of a rigid sacrificial substrate, e.g., a Si/SiO2 wafer, is advantageous when manufacturing a flexible probe, in order to provide sufficient support.

An exemplary wafer suitable for use in manufacturing of the probe 100 is schematically illustrated in **Fig. 2b****.**

Here, a 4" wafer is shown.

Once the fabrication process is accomplished, the probe 100 is detached from the sacrificial substrate, e.g., the wafer of **Fig. 2b****.**

Also, in the present embodiment, the method further comprises at least one passivation step.

In particular, said at least one passivation step may include interlayer passivation.

Additionally or alternatively, said at least one passivation step may include top passivation.

Additionally or alternatively, said at least one passivation step may include bottom passivation.

The method may further comprise a step of forming a second metal layer for at least one single-layer or double layer solution-gated graphene field effect transistor (SG-GFET) 14 over the second flexible encapsulation layer 24.

Then, a graphene preparation is prepared and transferred.

In the present embodiment, the method further comprises providing an additional top layer, such as the additional top layer 26 described above.

In the present embodiment, said additional top layer 26 is preferably made of SU-8.

**Fig. 3** shows a stimulation and control system 200 according to an embodiment of the invention.

In particular, the system 200 is adapted to implement closed-loop stimulation.

The system 200 comprises a control unit 202.

The system 200 further comprises a stimulation unit 204.

The control unit 202 and the stimulation unit 204 are operatively connected **(****Fig. 3****).**

In particular, the control unit 202 and the stimulation unit 204 may be configured to implement bi-directional communication (see arrow in **Fig. 3****).**

Communication between the control unit 202 and the stimulation unit 204 can be wireless.

Alternatively, communication between the control unit 202 and the stimulation unit 204 can be wired.

In particular, the control unit 202 is adapted to control operation of the stimulation unit 204, according to one or more stimulation parameters.

The stimulation unit 204 comprises at least one neural probe 100 as described in the foregoing.

Here, a stimulation protocol for the stimulation unit 204 can be triggered and/or controlled and/or adjusted by the control unit 202 based on one or more neural activities, detected through the SG-GFETs 14.

Thanks to the unique ability of SG-GFETs, e.g., single-layer or double-layer SG-GFETs, of recording full spectrum of brain activity (even in the infraslow activity range), the system 200 allows for effective treatment of a wide range of neurodegenerative pathologies, including neurological disorders (such as epilepsy or stroke) the biomarkers of which have proven to demonstrate themselves in infraslow frequencies.

**Fig. 2e** provides a schematic overview of the functioning of the closed-loop system 200.

In particular, the diagram shows an experimental implementation in a rodent (mice) model.

**Fig. 2f** provides a schematic representation of current to voltage conversion in a single-layer SG-GFET, as used in the neural probe 100 of the invention.

Full-bandwidth acquisition capabilities in a single-layer SG-GFET as used in the neural probe 100 of the invention are shown in **Fig. 2g****.**

### Neural probe characterization and recording performance

Laboratory tests were conducted by the inventors to characterize a neural probe according to the invention.

The results of these laboratory tests are illustrated in **Figs. 4a****-f.**

**Fig. 4a** shows individual transfer curves (see curves (i)) and transconductance (see curves **(ii))** of 16 single-layer SG-GFETs in one probe.

Here, thicker lines denote averages.

**Fig. 4b** shows CNP and transconductance values (for Vgs = CNP - 0.1V) dispersion statistics for 10 devices (16 transistors each). The results show good homogeneity of transistor characteristics.

**Fig. 4c** shows the results of cyclic voltammetry measurement in [-0.8, 0.8V] range, 50mV/s rate.

**Fig. 4d** shows impedance values at 10 and 1000 Hz for 10 devices.

Here, each device includes 14 rGO electrodes. The results show good homogeneity of electrode characteristics. rGO electrode impedances are very low in comparison to other technologies due to the porous nature of material. Low impedance provides excellent charge injection capacities.

**Fig. 4e** shows voltage response to current-controlled biphasic pulses of 500 µs per phase (see dashed lines) applied through rGO electrodes, 5 pulse amplitudes defined as a percentage of Amax = 210 µA.

**Fig. 4f** shows a map of the cathodic capacitive voltage excursion occurring at the interface between the rGO electrodes and an electrolyte during the injection of current pulses at different levels of injected charge and pulse widths.

Laboratory tests were also conducted by the inventors to define signal recording performance of the probe during simultaneous stimulation with rGO electrodes.

The results of these laboratory tests are illustrated in **Figs. 5a****-i.**

**Fig. 5a** illustrates the stimulation protocol used in the experiments with combinations of various stimulation parameters: pulse width (100 or 500 µs per phase), frequency (25, 130, 185 or 250 Hz), amplitude (10, 25, 50, 75 or 90 % Amax).

Here, each combination lasted ~40s.

**Fig**. **5b** shows the results of electrode (see line (i)) and transistor (see line (ii)) recordings during stimulation.

The last 3 pulses (250 Hz, 500 µs/phase, 90%A) in the stimulation protocol were mapped.

Stimulation was delivered through electrode C3.

**Fig. 5c** shows the results of voltage mapping by single-layer SG-GFETs.

**Fig**. **5d** provides a comparison of 500 µs pulse recorded by a single-layer SG-GFET and a rGO electrode, showing electrode amplifier saturation.

**Fig**. **5e** shows the results of transistor recording.

Here, 1000 stimulation pulses (F=250Hz, t=500us, A=90%) were overlapped.

**Fig**. **5f** shows a PSD plot of recording of a baseline and a stimulation period (F=250 Hz, t=500 µs, A=90%).

**Fig. 5g** provides a schematic overview of noise aliasing and signal quality dependence on sampling frequency.

**Fig. 5h** shows ratio of PSD plots from **Fig. 5f****.**

**Fig**. **5i** shows statistics of baseline normalized PSD value dependency on stimulation amplitude.

Here, 9 full stimulation protocols from **Fig**. **5a** were averaged.

**Fig.** 5j shows results of raw recording and recovery of 10Hz biosignal during stimulation.

Eventually, **Fig**. **5k** shows similar evidence as in **Fig**. **5i****,** but as a function of stimulation frequency.

In particular, **Fig. 5k** provides evidence that the recorded signal is not contaminated for a range of stimulation frequencies and amplitudes.

### In vivo experimental evidence

A series of *in vivo* experiments was carried out by the inventors using the neural probe of the invention, involving open-loop and closed-loop electrical stimulation triggered by real-time neural activity.

In a first set of experiments, an awake head-fixed rodent (mice) model was used to characterize the ability of the neural probe to accurately capture neural activity while simultaneously stimulating the brain.

In brief, recordings show how, below the stimulation frequency, activity perfectly resembles that which preceded stimulation, a finding which is further quantified in the "distortion factor" metric and presents robustness across multiple trials within and across animals.

**Figs. 6a****-h** show the results of *in vivo* stimulation artifact characterization.

**Fig. 6a** schematically shows a mouse on a Neurotar head-fixation system with a blow up of array position relative to ground and reference wires.

**Fig. 6b** shows the experimental timeline with picrotoxin chemoconvulsant injection.

**Fig. 6c** provides details of a stimulation paradigm.

**Fig. 6d** is a diagram illustrating time-frequency analysis showing appearance of epileptiform discharges following picrotoxin injection.

**Fig. 6e** shows time-frequency analysis of stimulation artifact.

**Fig. 6f** illustrates stimulation blow up with full band and filtered signals, showing remarkable resilience to stimulation artifacts.

**Fig. 6g** shows distortion factor before and during stimulation periods.

**Fig. 6h** shows distortion factor of 4 animals (namely, Animals 124 to 127), and different trials per animal with a probability distribution of the baseline values of the distortion factor.

In a second set of experiments, two rodent (mice) models were employed with the aim of demonstrating translational applications of the probe.

The first model consisted of focal Tsc1 knockout mimicking focal cortical dysplasia (i.e., a form of epilepsy).

This is achieved through a transgenic mouse model which has a flagged Tsc1 gene.

After injecting a viral vector in the emphasized area denoted with **(i)** in **Fig. 7a****,** the Tsc1 is knocked out in that region making it putatively hyperexcitable.

This serves as a way to assess if the probe is capable of delineating (or mapping) epileptic tissue from healthy brain areas, a common procedure during surgical brain resection of epileptic patients.

The second model consists of wild type mice who have been injected with a viral construct containing a form of opsins, i.e., a light sensitive protein.

This allows to fire neurons in a controlled manner with the use of specific light wavelengths.

A previous study demonstrated that it is possible to reliably induce infraslow activity (also called cortical spreading depolarizations) with optic stimulation.

The present experiment aims at demonstrating closed-loop capabilities of the probe to record infraslow activity and trigger stimulation.

This differs from the previous approach, where infraslow activity was induced with through stimulation.

The notion of modulating infraslow activity (i.e., detecting it and potentially changing it stimulation) is an aspect that also holds translational value.

These cortical spreading depolarizations are observed in different forms of paroxysmal disorders, such as migraines or epilepsy, and are known to be involved in sudden death in tested animals when they propagate to areas in the brainstem.

Therefore, modulating the way in which these events spread, could be a potential future application for the probe.

**Figs. 7a****-g** illustrate a proof of concept of *in vivo* applications.

**Fig. 7a** shows a detail of a focal epileptic mouse model with injection site of TSC1 knock out agent.

In the present experiment, the mouse was implanted with a neural probe according to the invention.

Here, the emphasized area denoted with (i) is putatively hyperexcitable.

**Fig. 7b** provides a demonstration of the hybrid array to map epileptiform tissue with stimulation off the hyperexcitable site not eliciting responses while stimulation over the excitable site does.

**Fig. 7c** shows a detail of wild-type mice with channelrhodopsin injection, to make the mouse reactive to optic stimulation.

**Fig. 7d** provides a demonstration of reliable induction of infraslow activity with optogenetics, detection with single-layer SG-GFETs, and stimulation with rGO electrodes.

**Fig. 5f** shows proof of propagation mapping of infraslow activity across the device.

Eventually, **Fig. 7g****,** shows the resulting change in direction due to stimulation in a row of rGO electrodes.

Single-layer SG-GFETs have proven capable of implementing reliable recording during stimulation over the entire frequency spectrum of brain activity, including infraslow frequencies.

Also, Single-layer SG-GFETs showed an appreciable level of artifact resilience.

The system is suitable for use in different *in vivo* applications, such as epileptic tissue mapping, CSD modulation and mapping, or epilepsy treatment.

### References

- 100: Neural probe

- 200: System

- 10: Substrate
- 12: Stimulation and recording array
- 14: Solution-gated graphene field effect transistor (SG-GFET)
- 16: Electrode, reduced-graphene oxide (rGO) electrode
- 18: Metal track (for the electrode(s))
- 20: Metal tracks (for the for the SG-GFET(s))
- 22: Encapsulation layer
- 24: Encapsulation layer
- 26: Additional top layer

- 202: Control unit (system)
- 204: Stimulation unit (system)

- T: Tip portion (of the neural probe)

## Claims

1. A stimulation and recording neural probe (100) at least comprising:
a substrate (10);
a stimulation and recording array (12) comprising:
at least one single-layer or double layer solution-gated graphene field effect transistor (SG-GFET) (14) for recording brain signals of a patient, and
at least one electrode (16) for applying electrical stimulation to a brain tissue and/or recording brain signals of the patient, and
metal tracks (18, 20) for the at least one electrode (16) and the at least one single-layer or double layer SG-GFET (14), encapsulated in respective first and second encapsulation layers (22, 24).

2. The neural probe (100) of claim 1,
**characterized in that**
the at least one electrode (16) is a reduced-graphene oxide (rGO) electrode (16).

3. The neural probe (100) of claim 1 or 2,
**characterized in that**
the substrate (10), the first encapsulation layer (22), and the second encapsulation layer (24) are made of a flexible material,
preferably wherein the first encapsulation layer (22), and the second encapsulation layer (24) are made of a polymeric material, a non-polymeric material, or a combination thereof,
more preferably wherein the first encapsulation layer (22) and the second encapsulation layer (24) are made of Polyimide or Parylene-C.

4. The neural probe (100) of any one of the preceding claims,
**characterized in that**
the stimulation and recording array (12) comprises a plurality of single-layer or double layer SG-GFETs (14) and rGO electrodes (16),
preferably wherein the stimulation and recording array (12) comprises an arrangement including rows of single-layer or double layer SG-GFETs (14) alternating with rows of rGO electrodes (16).

5. The neural probe (100) of claim 4,
**characterized in that**
the stimulation and recording array (12) comprises 16 single-layer or double layer SG-GFETs (14), and 14 electrodes (16).

6. The neural probe (100) of claims 4 and 5,
**characterized in that**
the stimulation and recording array (12) comprises an arrangement including four rows of single-layer or double layer SG-GFETs (14) alternating with four rows of electrodes (16),
wherein the last row of electrodes (16), formed at the bottom of the array (12), includes a reduced number of electrodes (16), preferably two electrodes (16).

7. The neural probe (100) of any of the preceding claims,
**characterized in that**
the at least one single-layer or double layer SG-GFET (14) has channel length between 1 × 1 µm to 1 x 1 mm, preferably of 50 x 50 µm.

8. The neural probe (100) of any of the preceding claims,
**characterized in that**
the at least one electrode (16) has diameter between 10 µm to 5 mm, preferably of 100 µm.

9. The neural probe (100) of any of the preceding claims,
**characterized in that**
the stimulation and recording array (12) is provided at a tip portion (T) of the neural probe (100).

10. The neural probe (100) of any of the preceding claims,
**characterized in that**
the neural probe (100) further comprises an additional top layer (26),
preferably wherein said additional top layer (26) is made of SU-8.

11. A method of manufacturing a stimulation and recording neural probe, the method at least comprising:
providing a substrate (10);
forming a first encapsulation layer (22) on said substrate (10);
forming a first metal layer for electrode tracks (18) on the first encapsulation layer (22);
defining and patterning at least one electrode (16) and the electrode metal tracks (18);
forming a second encapsulation layer (24);
defining and patterning VIAs;
defining and patterning the at least one single-layer or double layer SG-GFET (14) and SG-GFET metal tracks (20);
defining and patterning openings for the at least one electrode (16), and
defining and patterning an outline for the neural probe (100).

12. The method of claim 11,
**characterized in that**
the method further comprises at least one passivation step,
preferably wherein said passivation step includes:
interlayer passivation, and/or
top passivation, and/or
bottom passivation.

13. The method of any of claims 11 or 12,
**characterized in that**
the method further comprises:
forming a second metal layer for at least one single-layer or double layer solution-gated graphene field effect transistor (SG-GFET) (14) over the second encapsulation layer (24);
providing a graphene preparation and transferring said preparation on the first metal layer.

14. The method of any of claims 11 to 13, wherein the at least one electrode (16) is a reduced-graphene oxide (rGO) electrode (16),
**characterized in that**
before the step of defining and patterning at least one electrode (16) and the electrode metal tracks (18), the method further comprises:
filtrating a graphene oxide dispersion;
forming and transferring a graphene oxide membrane over the first metal layer.

15. The method of any of claims 11 to 14,
**characterized in that**
the substrate (10), the first encapsulation layer (22), and the second encapsulation layer (24) are made of a flexible material,
preferably wherein the first encapsulation layer (22), and the second encapsulation layer (24) are made of a polymeric material, a non-polymeric material, or a combination thereof,
more preferably wherein the first encapsulation layer (22) and the second encapsulation layer (24) are made of Polyimide or Parylene-C.

16. The method of any of claim 11 to 15,
**characterized in that**
the method further comprises providing an additional top layer (26),
preferably wherein said additional top layer (26) is made of SU-8.

17. A closed-loop system (200) for brain stimulation and recording, at least comprising:
a control unit (202), and
a stimulation and recording unit (204), operatively connected to the control unit (202), the stimulation unit (204) comprising at least one stimulation and recording neural probe (100) as defined in any of claims 1 to 11.
